# EUROPEAN PATENT SPECIFICATION

(11) **EP 0 862 440 B1**
(45) Date of publication and mention of the grant of the patent: **09.04.2003**
(21) Application number: 96928254.0
(22) Date of filing: 20.08.1996
(51) Int. Cl.: A61K 38/42, A61P 17/02

(54) **THERAPEUTIC USE OF HEMOGLOBIN IN PROMOTING WOUND HEALING**
THERAPEUTISCHE ANWENDUNG EINES HEMOGLOBINS ZUR FÖRDERUNG DER WUNDHEILUNG
UTILISATION THERAPEUTIQUE DE L'HEMOGLOBINE POUR FAVORISER LA CICATRISATION

(30) Priority: 26.10.1995 US 548667
(43) Date of publication of application: 09.09.1998
(73) Proprietor: BAXTER INTERNATIONAL INC., Deerfield, Illinois 60015 (US)
(72) Inventor: BURHOP, Kenneth, E., Mundelein, IL 60060 (US); LEPPANIEMI, Ari, K., Haartmaninkatu 4, 00290 Helsinki (FI); MALCOLM, Diana, S., Martinez, GA 30907 (US); SOLTERO, Raluan, La Jolla, CA 92037 (US)
(74) Representative: Bassett, Richard Simon
(86) International application number: US9613510
(87) International publication number: WO97015313

(56) References cited:
- US-A- 4 001 200
- US-A- 4 053 590
- US-A- 4 061 736
- US-A- 4 405 606
- US-A- 5 334 706
- US-A- 5 464 814
- AMERICAN JOURNAL OF CHINESE MEDICINE, 1986, Volume XIV, Nos. 3-4, MAHDIHASSAN S., "Blood as the Earliest Drug, Its Substitutes, Preparations and Latest Position", pages 104-109.

## Description

### BACKGROUND OF THE INVENTION

Trauma related wounds with substantial incidental blood loss constitute one class of medical emergency requiring supportive intervention and follow-up treatment to facilitate proper healing. The typical emergency intervention involves transfusions of blood or plasma expanders such as lactated Ringer's solution. Where blood loss is extreme, it is critically important to replace blood volume quickly to prevent circulatory collapse and organ damage.

Another group of wounds are deliberately inflicted during operative procedures. Some surgeries such as hip replacement, aortic repair, or organ transplants involve substantial losses of blood. Fluid replacement requiring transfusion of several units of whole blood in addition to infusion of other fluids is not uncommon. Complete healing of surgical incisions may take several weeks.

Wound healing or wound repair involves a complex series of cellular, biochemical, and physiological events. Many conditions complicate the repair process. It has been noted that blood loss frequently associated with the wound itself, is a significant complication, and prolongs the healing process. McGinn, Br. J. Surg., 63: 742 (1976) demonstrated that blood loss insufficient to cause clinical shock can give rise to impaired wound healing. Subsequently, in carefully controlled rat studies, Taylor, et al., Br. J. Surg., 74: 316 (1987) showed that significant impairment of laparotomy wounds occurs after loss of about ten percent of the circulating blood volume.

It would therefore be anticipated that patients undergoing surgery, or persons suffering trauma, resulting in blood loss not fully compensated by transfusion, would experience some impairment in wound healing. In experimental models, the integrity of the repaired wound may be measured by the amount of physical force required to rupture the wound interface. See, for example, Greenwald, et al., Plast. and Reconst. Surg., 91: 1087 (1993). Thus, there exist in the literature test models for assessing the extent of wound healing impairment resulting from blood loss, and the therapeutic value of replacement fluids in reversing anticipated impairment.

An understanding of the process of wound repair is set out in detail in The Molecular and Cellular Biology of Wound Repair, eds. Clark, et al. Plenum Press: N.Y. (1987), and in a clinical context, Zitelli, J., Adv. Dermatol., 2: 243 (1987). The process begins with infiltration of neutrophils and continues through a series of defined, but overlapping stages. From a cellular standpoint, neutrophils are succeeded by macrophages, which are necessary for normal healing, and finally fibroblast profileration. The orchestration of the successive stages of repair including, inflammation, re-epithelialization, fibroplasia (wound contraction), neovascularization, matrix formation, and wound remodelling are mediated by a complex array of secreted factors, blood and immune components, hormones, and architectural substances.

The puzzling aspect of this process, is that while all of these stages are reproducibly observed, animals genetically deficient in some cellular or soluble components may, nevertheless, exhibit normal endpoint healing. For example, neutropenic animals heal normally, but animals deficient in polymorphonuclear cells do not. Some substances, such as fibronectin are required for critical cell recruitment, whereas others have no defined role, and may be gratuitously released.

In the treatment of wounds several strategies have been developed to facilitate or enhance the repair process. The oldest of these are surface debridement, use of topical disinfectants, and wound dressings. More sophisticated approaches include hydrogel dressings, use of electrical stimulation and ultrasound, and hyperbaric oxygen. For a comprehensive review of wound treatment, see Kloth, et al., Wound Healing: Alternatives in Management, F.A. Davis: Philadelphia (1990).

### SUMMARY OF THE INVENTION

Medical intervention to reverse the deleterious effect of blood loss on wound repair has the potential to shorten recovery time from trauma or surgery, and reduce the number and severity of complications. It is therefore an object of the present invention to provide a systemically or intravenously administered fluid-based drug which restores wound healing efficiency. The present method is based on the observation that administering stroma-free, pyrogen-free hemoglobin in a therapeutic amount enhances wound repair after significant blood loss.

The method of treating wounds, promoting wound repair, and enhancing wound repair or healing comprises administering pharmaceutically compatible hemoglobin (stroma- and pyrogen-free) in an amount (range of about 50-2500 mg/kg of body weight) necessary to elevate mean arterial blood pressure to within 80 percent or more of base value, or wherein no hypotension occurs to a value at least 105 percent of base value. The hemoglobin may be diaspirin crosslinked, or crosslinked by other methods to afford structural and conformational stability.

Administration of the hemoglobin according to the present method is preferably by intravenous infusion peritraumatically or perioperatively either in a single dose given within the first twenty four hours post-trauma or post-operation, or alternatively, in multiple subdoses over a twenty-four hour period of time.

The method of comprising wound repair further comprises administering perioperatively or peritraumatically hemoglobin, preferably diaspirin crosslinked hemoglobin, to a patient having no other medical indication for administering hemoglobin or any other resuscitative fluid, i.e., having undergone subclinical blood loss.

### DETAILED DESCRIPTION OF THE PREFERRED EMBODIMENT

In the present invention, hemoglobin is administered perioperatively in surgical procedures or peritraumatically following significant or subclinical blood loss, in a therapeutically effective amount to enhance or promote the wound healing process. Subclinical blood loss means a loss of blood too insignificant to require medical intervention, as by administration of resuscitative fluid such as whole blood or fractions, plasma expanders, synthetic compounds, and hemoglobins. This beneficial result is unexpected in light of literature reports that suggest impairment of wound healing is associated with anoxia or ischemic resulting from the severe depression in oxygen delivery subsequent to severe blood loss. Temporal relief of oxygen delivery in the first twenty four hours by hemoglobin administered in relatively small doses cannot replace the totality of oxygen carrying capacity restored upon transfusion of whole blood. The hemoglobin is effectively eliminated from the blood stream in about 72 hours.

Pryzbelski (U.S. Pat. No. 5,334,706) discloses that administration of hemoglobin increases tissue perfusion to a greater extent than would be expected from the level of additional oxygen carrying capacity contributed by the hemoglobin. In Pryzbelski, the pharmacologic effect is exerted very close in time to the ischemic event the hemoglobin is being administered to relieve, and the resultant tissue damage which is to be averted. However, in the present invention, the beneficial effect on wound repair is only observed after one week post-administration. Since the cellular and biochemical events involved in the various stages of wound healing overlap, it is impossible for Applicants to pinpoint the step or steps in the process which are affected by the treatment.

The hemoglobin is administered perioperatively or peritraumatically. This means the full dose should be given within the first 24 hours after the wound occurs. The hemoglobin may be given in one dose in an amount ranging from 50 to 2500 mg/kg of body weight. Alternatively, that dose may be administered in that time period by slow continuous infusion or in a series of small subdoses. The preferred route of administration is intravenous infusion, although arterial cannulization may be carried out in appropriate clinical circumstances.

The present method may be utilized in any circumstance wherein blood loss occurs in the context of tissue damage. This includes those instances in which the situs of primary blood loss is not the lesion to be treated. For example, the present method is indicated in the situation in which there is blood loss from internal injuries, but the principal lesions are burns. Thus, the present method encompasses the administration of hemoglobin to a mammal in the range of 50 to 2500 mg/kg of body weight in the treatment of a lesion accompanied by a substantially simultaneous significant or subclinical blood loss. A clinically significant blood loss can be as little as 2-3% of the total estimated blood volume, depending on the overall medical condition of the patient. Blood loss of lesser volume is deemed to be subclinical.

The actual dose given is dictated by the comprehensive clinical presentation of the particular patient. There will be those medical situations known to those in the healing arts, in which the circumstances of acute blood loss indicate immediate large dose administration of hemoglobin, as direct replacement therapy for blood loss. Eliminating the life threatening aspect of the case is paramount to the secondary benefit of enhanced wound repair should the patient survive. In the most preferred embodiment of the present method, an infusion of hemoglobin in a therapeutically safe dose range (50 to 2500 mg/kg of body weight) may be given to patients who tolerated an operative procedure or underwent trauma without the need for transfusion of blood or infusion of other resuscitative fluids.

The hemoglobin utilized in the present invention may be of any type which is stroma-free and modified chemically to prevent subunit dissociation and to increase the oxygen binding affinity to the range of P₅₀, values between about 20 and 45 mm Hg. The modified hemoglobin may be a conjugated hemoglobin, crosslinked hemoglobin, or polymerized hemoglobin.

Several examples of hemoglobin modification technology have been described in the scientific literature which may be used to advantage in the practice of the present invention. For example, see the review contained in Winslow, R.M., Hemoglobin-based Red Cell Substitutes, The John Hopkins U. Press (1992). More specifically, the methods of making chemically modified hemoglobin are set forth hereinafter.

A conjugated hemoglobin is one to which a non-protein macromolecule is bound covalently to hemoglobin. One example is a hemoglobin chemical modified by polyalkylene glycol, which is described together with a process for its preparation in WO 9107190 (Enzon). An example of a hemoglobin conjugated to poly(alkylene oxide) and a process for its preparation are provided in U.S. Patent Nos. 4,301,144, 4,412,989 and 4,670,417, and in Japanese Patent Nos. J59,104,323 and J61,053,223 (Ajinomoto). Hemoglobin may be conjugated to inulin in a process disclosed in U.S. Patent No. 4,377,512 (Ajinomoto).

A crosslinked hemoglobin contains an intramolecular chemical link. Examples of crosslinked hemoglobins and methods for their preparation are described in U.S. Patent Nos. 4,001,401 and 4,053,590, which disclose intramolecular crosslinking between an alpha and beta subunit of a hemoglobin tetramer utilizing compounds such as halogenated cycloalkanes, diepoxides, and diazobenzidines. In the present method, a preferred modified hemoglobin is crosslinked with bis(3,5-dibromosalicyl)fumarate to create a fumarate crosslink between the two alpha subunits. This crosslinked hemoglobin is more fully described, together with methods for its preparation, in U.S. Patent Nos. 4,598,064. 4,600,531, RE 34,271, omitting the chromatography step. It is preferably manufactured under the conditions disclosed in U.S. Patent No. 5,128,452 (Hai) to prevent crosslinking between beta and chains. WO 9013309 (Staat Der Nederlanden De Minister Van Defeuric) discloses a method for crosslinking hemoglobin through a β-β linkage. The preferred diaspirin crosslinked hemoglobin will hereafter be referred to as "DCLHb".

A polymerized hemoglobin is one in which intermolecular crosslinking of hemoglobin tetramers has been used to increase the molecular weight of the modified hemoglobin. An example of a polymerized hemoglobin and a process for its preparation are described in U.S. pending applications Serial Nos. 8/149,679, 8/173,882, 8/480,593 and 8/473,459. U.S. Patent No. 4,777,244 discloses a method for crosslinking and polymerizing with aliphatic dialdehydes.

A hemoglobin that has been modified by a combination of methods is exemplified by the following. Hemoglobins modified by pyridoxal-5'-phosphate to adjust the oxygen affinity and by polyethylene glycol conjugation and processes for its preparation are described in Japanese Patent Nos. J59,089,629, J59,103,322 and J59,104,323 (Ajinomoto). U.S. Patent No. 5,248,766 discloses a crosslinking polymerizing strategy and a process for covalently interconnecting tetrameric units with oxiranes to form polyhemoglobins with molecular weights in excess of 120,000 Daltons. The foregoing patents disclosing polymerized hemoglobins, (see U.S. Patent Nos. 5,194,590, 5,248,766, Japanese Patent Nos. J59,103,322, J59,089,629 and J59,104,323).

Hemoglobin may be modified by site-directed mutagenesis and expressed in micro-organisms or transgenic animals. Recombinant mutant and artificial hemoglobin and its production in cell cultures or fluids is described in U.S. Patent 5,028,588 (Somatogen). Di-alpha and di-beta globin-like polypeptide(s) used for production of hemoglobin in bacteria and yeast are described in WO 9013645 (Somatogen). A non-natural multimeric hemoglobin-like protein is described in WO 9309143 (Somatogen). In general any method of crosslinking, polymerizing, or genetically modifying, or combination thereof which yields a base tetramer having a P₅₀ in the operative range of 20 to 45 mm Hg will have efficacy in the present method. Conditions may be adjusted for each such crosslinked tetramer or polymer derived therefrom without undue experimentation. Further advantages of the present invention will be apparent from the Example which follows.

### Example

Male Sprague-Dawley rates were anesthetized intraperitoneally with pentobarbital (35-45 mg/kg) and intramuscularly with Ketamine (60-90% mg/kg). The neck and back of each animal was shaved and prepped with chlorhexidence solution. A transverse incision was made in the neck, and the internal jugular vein and carotid artery were catheterized using polyethylene catheters. Then a full thickness skin wound (including panniculus carnusus) was created with the scalpel in the dorsal (back) midline from the interscapular region to the midpelvic level. The wound was covered with a wet gauze and the animal then was connected to the Buxco physiograph to monitor Mean Arterial Pressure, Heart Rate and pulse pressure. Animals were bled 15 ml/kg at a rate of 1 ml/min. 45 minutes later the animals were resuscitated with either 15 ml/kg of 10 g% DCLHb or shed blood or not resuscitated. A fourth group of animals (SHAM) were wounded but not bled and severed as controls, 1:1 shed blood (15 ml/kg), not resuscitated (NR), or sham treated (wounded but not bled). The catheters were then removed and both the carotid and jugular vein were tied to prevent any further bleeding. The neck incision was closed with a running stitch of 3.0 nylon 60 minutes post injury. Then the 6 cm incision in the dorsum of the rat was closed with interrupted simple sutures of 6.0 nylon spaced at 0.5 intervals. The animals were allowed to recover from anesthesia and surgery and returned to their cages for observation.

Tables 1A and 1B give the blood pressure data for animals sacrificed at one and three weeks respectively for each of the DCLHb, Sham, and blood restored groups. As shown in previous studies, the DCLHb treated group showed uniformly elevated mean arterial blood pressures.

**TABLE 1A**

| Treatment | Animal # | Harvest | Map Start | Map End | Map Low |
|---|---|---|---|---|---|
| DCLHb | 5239511 | 1 wk' | 125 | 144.4 | 39.1 |
| DCLHB | 5039510 | 1 wk' | 134.1 | 140.1 | 57.6 |
| DCLHb | 5039512 | 1 wk' | 138.2 | 95.2 | 59.6 |
| DCLHb | 5039509 | 1 wk' | 108.3 | 135.2 | 41.5 |
| DCLHb | 4119501 | 1 wk' | 108.6 | 145.1 | 46.2 |
| DCLHb | 4119502 | 1 wk' | 140 | 160.4 | 43.3 |
| DCLHb | 4059502 | 1 wk' | 116.3 | 144.1 | 53.8 |
| DCLHb | 4059501 | 1 wk' | 117.6 | 157.5 | 60 |
| DCLHb | 4049501 | 1 wk' | 160.7 | 138.6 | 50.5 |
| DCLHb | 409502 | 1 wk' | 127 | 168.4 | 49.9 |
| Sham | 5029508 | 1 wk' | 117.7 | 103.2 | |
| Sham | 5039501 | 1 wk' | 146 | 96.2 | |
| Sham | 5039505 | 1 wk' | 124.2 | 113.5 | |
| Sham | 5029505 | 1 wk' | 123.3 | 101.3 | |
| Sham | 4049506 | 1 wk' | 123.1 | 116.2 | |
| Sham | 4049503 | 1 wk' | 116.6 | 98 | |
| Sham | 4049505 | 1 wk' | 127.3 | 99.8 | |
| Sham | 4049504 | 1 wk' | 116.3 | 1.20 | |
| Sham | 4039501 | 1 wk' | 117.3 | 90.4 | |
| Sham | 2239502 | 1 wk' | 123.9 | 104 | |
| Blood | 5039508 | 1 wk' | 124.9 | 98.8 | 63.9 |
| Blood | 4179502 | 1 wk' | 132.4 | 120 | 42.1 |
| Blood | 4179504 | 1 wk' | 132,9 | 104 | 51.5 |
| Blood | 4179503 | 1 wk' | 112.7 | 117.5 | 45.5 |
| Blood | 4129506 | 1 wk' | 138.3 | 107.9 | 83.1 |
| Blood | 4129502 | 1 wk' | 135.3 | 110.8 | 56.6 |
| Blood | 4129501 | 1 wk' | 139.2 | 121.3 | 62.5 |

**TABLE 1B**

| Treatment | Animal # | Harvest | Map Start | Map End | Map Low |
|---|---|---|---|---|---|
| DCLHb | 5199501 | 3 wk' | 120.1 | 140.7 | 60.5 |
| DCLHB | 4279501 | 3 wk' | 125.4 | 141.1 | 42.9 |
| DCLHB | 4279503 | 3 wk' | 120.3 | 118 | 43.8 |
| DCLHb | 4279505 | 3 wk' | 127.1 | 140.8 | 35 |
| DCLHb | 3159602 | 3 wk' | 113.6 | 126.8 | 46.8 |
| DCLHB | 3159501 | 3 wk' | 130.5 | 136.5 | 42 |
| DCLHb | 3029502 | 3 wk' | 134.3 | 151.6 | 43 |
| DCLHb | 3019501 | 3 wk' | 137.7 | 154 | 24.8 |
| DCLHb | 3019503 | 3 wk' | 143.2 | 128 | 87.9 |
| DCLHb | 2279501 | 3 wk' | 132.7 | 125.8 | 43.3 |
| Sham | 4289505 | 3 wk' | 130.6 | 87.3 | |
| Sham | 4289508 | 3 wk' | 145.6 | 121.6 | |
| Sham | 4289507 | 3 wk' | 122.3 | 80.1 | |
| Sham | 4289506 | 3 wk' | 105.4 | 99.7 | |
| Sham | 2039502 | 3 wk' | 117 | 110 | |
| Sham | 2159501 | 3 wk' | 127.8 | 100.1 | |
| Sham | 2109501 | 3 wk' | 136.7 | 134 | |
| Sham | 1209502 | 3 wk' | 113 | 116 | |
| Sham | 2039501 | 3 wk' | 126.3 | 127.9 | |
| Sham | 1209502 | 3 wk' | 109.7 | 101 | |
| Blood | 4289501 | 3 wk, | 105.6 | 100.3 | 60.5 |
| Blood | 4289502 | 3 wk' | 111 | 101.3 | 35.9 |
| Blood | 4289509 | 3 wk' | 127.9 | 77.6 | 28 |
| Blood | 4289504 | 3 wk' | 131.7 | 100.1 | 47.6 |
| Blood | 3299502 | 3 wk' | 135.7 | 100.8 | 49.1 |
| Blood | 3299501 | 3 wk' | 141 | 110 | 77.9 |
| Blood | 3279501 | 3 wk' | 142.3 | 84.4 | 40.5 |
| Blood | 3279502 | 3 wk' | 115.1 | 108.1 | 47.6 |
| Blood | 3279502 | 3 wk' | 112 | 93.5 | 29.8 |

Half the animals from each group were sacrificed after 1 week, the remainder after 3 weeks. Immediately following sacrifice, the dorsal skin was removed (full-thickness, including panniculus carnosus) to include the entire wound. The skin was trimmed and cut into strips 0.5 x 2.0 cm oriented perpendicular to the long axis of the wound. Sutures were removed from each strip before testing. The specimens were placed in a Tensiometer (John Chatillon and Sons, New York, N.Y.) and held in nonslip jaws and stretched apart at a constant speed (50 mm/min) until rupture. The rupture force or wound breaking strength (WBS) was recorded and compared between groups to determine rate of wound healing.

Mann-Whitney Rank Sum Test or t test was used for statistical analysis with significance at p<0.05 level. Data is presented as mean±SEM. Sham means wounded but not hemorrhaged. The results are set forth in Table 2.

**TABLE 2**

| TREATMENT (n)=6) | WBS 1 WK (kg/0.05 cm) | WBS 3 WKS (kg/0.05 cm) |
|---|---|---|
| Sham | 0.07±0.01 | 1.65±0.09* |
| DCLHb | 0.05±0.04 | 1.25±0.09$# |
| Blood | 0.07±0.02 | 0.99±0.07 |
| NR | 0.04±0.01 | 1.02±0.04 |
| P<0.05 *compared to all others, to $blood and to #NR (not resuscitated). | | |

WBS was not significantly different at 1 wk between groups. In conclusion moderate blood loss impaired wound healing in this rat model. Wound breaking strength was greater in DCLHb-treated rats than in blood-treated rats, suggesting that DCLHb may be superior to blood in improving wound healing in patients requiring transfusions after intraoperative hemorrhage.

## Claims

1. Use of stroma free, pyrogen free haemoglobin for the manufacture of a therapeutic preparation for promoting or enhancing wound repair in a patient who has tolerated an operative procedure, or undergone trauma, without transfusion of blood or other resuscitative fluid.

2. Use according to Claim 1 in a preparation, which is intravenously administrable.

3. Use according to Claim 1 or 2, for enhancing wound repair following subclinical blood loss

4. Use according to any preceding claim, wherein the preparation contains haemoglobin in an amount from 50 to 2500 mg/kg of body weight of the patient.

5. Use according to Claim 4, for administration within 24 hours after the wound occurs.

6. Use according to any preceding claim, wherein the haemoglobin is chemically modified.

7. Use according to Claim 6, wherein the chemically modified haemoglobin is selected from conjugated haemoglobin, crosslinked haemoglobin, or polymerized haemoglobin.

8. Use according to any preceding claim, wherein the haemoglobin is selected from recombinant mutant and artificial haemoglobin.

## Patentansprüche

1. Verwendung von stromafreiem, pyrogenfreiem Hämoglobin für die Herstellung eines therapeutischen Präparats zur Förderung oder Beschleunigung der Wundheilung bei einem Patienten, der operiert worden ist oder eine Verletzung erlitten hat, ohne die Transfusion von Blut oder einem anderen Wiederbelebungsfluid.

2. Verwendung nach Anspruch 1
in einem Präparat, das intravenös verabreicht werden kann.

3. Verwendung nach Anspruch 1 oder 2
zur Beschleunigung der Wundheilung nach einem subklinischen Blutverlust.

4. Verwendung nach einem der vorstehenden Ansprüche,
wobei das Präparat Hämoglobin in einer Menge von 50 bis 2500 mg
pro kg Körpergewicht des Patienten enthält.

5. Verwendung nach Anspruch 4
zur Verabreichung innerhalb von 24 Stunden nach Auftreten der Verwundung.

6. Verwendung nach einem der vorstehenden Ansprüche,
wobei das Hämoglobin chemisch modifiziert ist.

7. Verwendung nach Anspruch 6,
wobei das chemisch modifizierte Hämoglobin aus konjugiertem Hämoglobin, vernetztem Hämoglobin oder polymerisiertem Hämoglobin ausgewählt ist.

8. Verwendung nach einem der vorstehenden Ansprüche,
wobei das Hämoglobin aus rekombinantem mutiertem und künstlichem Hämoglobin ausgewählt ist.

## Revendications

1. Utilisation d'hémoglobine apyrogène exempte de stroma pour la fabrication d'une préparation thérapeutique pour favoriser ou renforcer la cicatrisation chez un patient qui a subi une intervention chirurgicale, ou qui a subi un trauma, sans transfusion de sang ou d'un autre fluide de réanimation.

2. Utilisation selon la revendication 1 dans une préparation qui peut être administrée par voie intraveineuse.

3. Utilisation selon la revendication 1 ou 2 pour renforcer la cicatrisation après une perte de sang subclinique.

4. Utilisation selon l'une quelconque des revendications précédentes, dans laquelle la préparation contient de l'hémoglobine en une quantité de 50 à 2500 mg/kg de poids corporel du patient.

5. Utilisation selon la revendication 4 pour une administration dans les 24 heures suivant l'apparition de la plaie.

6. Utilisation selon l'une quelconque des revendications précédentes, dans laquelle l'hémoglobine est chimiquement modifiée.

7. Utilisation selon la revendication 6, dans laquelle l'hémoglobine chimiquement modifiée est choisie parmi l'hémoglobine conjuguée, l'hémoglobine réticulée ou l'hémoglobine polymérisée.

8. Utilisation selon l'une quelconque des revendications précédentes, dans laquelle l'hémoglobine est choisie parmi l'hémoglobine mutante recombinée et l'hémoglobine artificielle.
